# EUROPÄISCHE PATENTSCHRIFT

(11) **EP 0 880 352 B1**
(45) Veröffentlichungstag und Bekanntmachung des Hinweises auf die Patenterteilung: **17.11.1999**
(21) Anmeldenummer: 98905128.9
(22) Anmeldetag: 03.02.1998
(51) Int. Cl.: A61K 31/485, A61K 47/02, A61K 47/06

(54) **STABILISIERUNG VON NALOXONHYDROCHLORID**
STABILIZATION OF NALOXONE HYDROCHLORIDE
STABILISATION DE CHLORHYDRATE DE NALOXONE

(30) Priorität: 14.02.1997 DE 19705537
(43) Veröffentlichungstag der Anmeldung: 02.12.1998
(73) Patentinhaber: GÖDECKE AKTIENGESELLSCHAFT, D-10587 Berlin (DE)
(72) Erfinder: Raffelsberger, Bernd, 79194 Gundelfingen (DE)
(74) Vertreter: Mansmann, Ivo
(86) Internationale Anmeldenummer: EP9800556
(87) Internationale Veröffentlichungsnummer: WO9835679

(56) Entgegenhaltungen:
- WO-A-97/33566
- DE-A- 4 423 850
- DATABASE WPI Section Ch, Week 9550 Derwent Publications Ltd., London, GB; Class A96, AN 95-390229 XP002067438 & JP 07 267 862 A (SEKISUI CHEM IND CO LTD) , 17.Oktober 1995
- DINGLEY A.L. ET AL: "Narcan Inhibition of Human Liver Alcohol Dehydrogenase" BIOCHEMICAL AND BIOPHYSICAL RESEARCH COMMUNICATIONS, Bd. 131, Nr. 1, 1985, Seiten 299-306, XP002067436
- HATCH R.C. ET AL: "Effects of Various Known and Potential Cyanide Antagonists and a Glutathione Depletor on Acute Toxicity of Cyanide in Mice" VETERINARY AND HUMAN TOXICOLOGY, Bd. 32, Nr. 1, 1990, Seiten 9-16, XP002067437

## Beschreibung

Die vorliegende Erfindung betrifft ein Verfahren zur Stabilisierung von Naloxon in Lösungen, besonders in sauren wässrigen Lösungen, sowie in festen oder halbfesten Stoffgemischen, insbesondere Arzneimitteln. Die Erfindung betrifft weiterhin Stoffgemische, insbesondere Arzneimittelformen, welche einen Stabilisator enthalten, der die Dimerisierung des Naloxons zu Bisnaloxon verhindert.

Naloxon, ((-)12-allyl-7,7a,8,9-tetrahydro-7,3a-dihydroxy-4aH-8,9c-iminoethanophenathro[4,5-bcd]furan-5,6H-on) ist ein Morphinantagonist (Narcanti®) der Formel 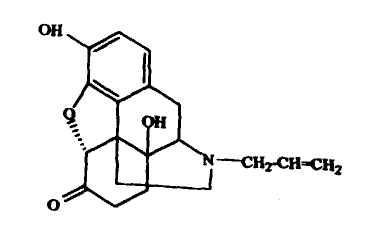 aus der Gruppe der Phenanthrenalkaloide.

Bisher ist man davon ausgegangen, daß Naloxon und seine Salze wie z.B das Hydrochlorid ziemlich stabile Verbindungen sind, die auch in sauren Lösungen und unter dem Einfluß von Radikalbildnern wie z.B. Sauerstoff keiner nennenswerten Zersetzung (Oxidation, Dimerisierung, Umlagerung etc.) unterliegen.
Neuere Langzeituntersuchungen haben jedoch gezeigt, daß Naloxon, im Gegensatz zur etablierten Ansicht der Fachwelt, ein Stoff ist, der unter ungünstigen Bedingungen z.B. solche, die die Radikalbildung unterstützen, insbesondere zu unerwünschten intramolekularen Reaktionen neigt, aber auch mit Begleitstoffen reagieren kann. Der chemische Ablauf dieser Reaktionen ist noch nicht genauer untersucht, so daß deren Verhinderung sich vorerst auf empirische Ansätze und Versuche stützen muß.

Es wird derzeit angenommen, daß sich in einer selektiv verlaufenden Reaktion gemaß Formelschema I dimere Naloxon-derivate und hierbei insbesondere das 2,2'-Bisnaloxon bilden. Diese Reaktion wird nach unveröffentlichten Untersuchungen durch in der Lösung zusätzlich anwesende, stickstoffhaltige organische Verbindungen begünstigt. Ausgelöst wird diese Dimerisierung wahrscheinlich durch oxidierend wirkende Stoffe und/oder Radikale, die in geringen Mengen immer vorhanden sind. Die spontan und selektiv verlaufende Bildung von

Bisnaloxon in Arzneimitteln ist in der Literatur bisher nicht beschrieben und ist speziell in wäßriger, saurer Lösung überraschend, da die Bildung dimerer Verbindungen verwandter Substanzklassen in der Regel ziemlich drastische Reaktionsbedingungen und alkalisches Milieu erfordert, bzw. die Anwendung von stark oxidierend wirkenden Enzymen verlangt. Die Existenz eines Naloxon-Dimeren ist zwar an sich bekannt (US-Pharmakopeia, National Formulary, Supplement, 15. November 1996, page 3434).
Die dort enthaltene Vorschrift zur Wirkstoffprüfung empfiehlt, Naloxonhydrochlorid vor seinem Einsatz in Injektionslösungen auf die Anwesenheit von 2,2'-Bisnaloxon, zu untersuchen.
Die Veröffentlichung sagt jedoch nichts aus über die Bildung von Bisnaloxon im fertigen Arzneimittel und die Lagerstabilität von Arzneimittelzubereitungen mit dem Wirkstoff Naloxon.

Aus WO 97/33566 ist es bereits bekannt, bestimmten Arzneimitteln, die neben anderen Stoffen auch Naloxon enthalten können, Antioxidantien zuzugeben. Es ist jedoch der Druckschrift nicht zu entnehmen, was die Antioxidantien bewirken sollen, insbesondere ist nichts über die Dimerisationsneigung des Naloxons gesagt.

Aus der DE 44 23 850 ist es bekannt, die Farbstabilität des Pflasters einer transdermalen Naloxonzubereitung mittels Dimethylsulfoxid oder Ascorbylpalmitat zu verbessern. Eine potentielle Stabilisation von Naloxon durch Verhinderung von dessen Dimerisation beschreibt das Dokument nicht.

Aus Biochemical and Biophysical Research Communications Bd. 131, Nr. 1, 1985, Seiten 299 - 306, XP002067436, ist es bekannt, daß Narcan®, eine Naloxon enthaltende parenterale Zubereitung, Methyl- und Propylparaben als Konservierungsmittel enthält. Das Dokument sagt nichts über eine potentielle Verhinderung der Dimerisierung von Naloxon aus.

Aufgabe der vorliegenden Erfindung ist es, die unerwünschte intramolekulare Umsetzung zu Bisnaloxon, aber auch die intermolekularen Reaktionen des Naloxons mit Begleitstoffen, zu verhindern und somit das Naloxon und dessen Salze insbesondere als Wirkstoff in festen und flüssigen Arzneimittelformen wirksam zu stabilisieren.

Zur Lösung dieser Aufgabe wurden zunächst Modellreaktionen entwickelt, die (ebenso wie die spontan ablaufende Dimerisierung von Naloxon) zu Bisnaloxon führen. Im Gegensatz zur spontan ablaufenden Dimerisierung, die unter Stressbedingungen in der Regel nach Ablauf von einigen Wochen beobachtet werden kann, sollten diese Modellreaktionen im Zeitrahmen von einigen Stunden bis Tagen in gleicher Weise ablaufen. In einem zweiten Schritt wurde dann untersucht, auf welche Weise die induzierten Nebenreaktionen möglichst quantitativ unterdrückt werden können.

Als geeignete Modellreaktionen haben sich das mehrstündige Erhitzen von salzsauren Naloxonhydrochloridlösungen auf 70°C, die Oxidation der Lösung mit einer verdünnten Kaliumpermanganatlösung im sauren Bereich, die Oxidation mit einer Aufschlämmung von Eisen(III)oxid in salzsaurer Lösung, das Erhitzen der Lösung in Gegenwart von Azobisisobutyronitril, sowie das Bestrahlen einer azobisisobutyronitrilhaltigen Naloxonhydrochloridlösung mit intensivem Tageslicht erwiesen. Alle Reaktionen führten zunächst sehr selektiv zur Bildung von Bisnaloxon in Mengen von ca. 5 - 10%, die Bestrahlung der Lösung ergab bis zu 40% Bisnaloxon. Bei längerer Dauer der Reaktion und unter drastischeren Bedingungen traten dann naturgemaß auch andere Umwandlungsprodukte des Naloxonhydrochlorids auf.

Im Rahmen der umfangreichen Untersuchungen wurde die inhibierende Wirkung einer ganzen Reihe von Substanzen geprüft. Diese Substanzen wurden naloxonhydrochloridhaltigen Lösungen in definierten Mengen zugesetzt und die jeweils erhaltenen Mischungen einer oder mehreren Modellreaktionen unterworfen.

Zunächst wurden typische Radikalfänger bzw. Antioxidantien eingesetzt. Die Wirksamkeit der Inhibitoren wurde anhand der verzögerten bzw. nicht beobachteten Bildung von Bisnaloxon getestet. Zur Quantifizierung von Bisnaloxon in naloxonhydrochloridhaltigen Lösungen wurden speziell hierfür geeignete HPLC-Methoden und HPTLC-Methoden entwickelt.

Als überraschend bereits in äußerst geringen Konzentrationen als Stabilisatoren wirksam erwiesen sich Antioxidantien wie Schwefeldioxid, Natriumsulfit, Natriumbisulfit, Ascorbinsäure und deren Derivate und Tocopherol sowie dessen wasser- und fettlöslichen Derivate wie z. B. Tocofersolan® oder Tocopherolacetat. Aber auch Sulfite, Bisulfite und Hydrogensulfite von Kalium-, Calcium und anderen Metallen zeigen eine gute, die Dimerisierung inhibierende Wirkung.

Erstaunlicherweise waren aber auch Verbindungen wirksam, deren antioxidative und Radikalfängerwirkung sonst kaum zum Tragen kommt oder überhaupt nicht bekannt ist:
PHB-Ester, BHA, BHT, Gallate sowie niedere Fettsäuren, wie Ameisen-, Essig-, und Propionsäure, Fruchtsäuren, wie z.B. Äpfel-, Fumar-, Milch-, Citronen-, und Weinsäure, aber auch Phosphorsäuren wie z.B. Orthophosphorsäure, Sorbin- und Benzoesäure sowie deren Salze, Ester, Derivate und isomere Verbindungen, Ascorbylpalmitat, Lecithine, ein- und mehrfach hydroxylierte Benzolabkömmlinge, wie z.B. Phenol, Hydrochinon oder Kresol, Ethylendiamintetraessigsäure und deren Salze, Citraconsäure, Cystein, L-Cystin, Conidendrine, Diäthylcarbonate, Methylendioxyphenole, Kephaline, β,β'-Dithiopropionsäure, Biphenyl und andere Phenylderivate. Schwach inhibierend sind Salze der Salpeter- und salpetrigen Säure.

Durch Zugabe der angegebenen Inhibitoren insbesondere von Schwefeldioxid, schwefligen Säuren oder von deren Alkali- oder Erdalkalisalzen oder von Ethylendiamintetraessigsäure oder deren Salzen in den geeigneten Konzentrationen, die sich mittels der beschriebenen Kurztests für die jeweilige Zusammensetzung rasch und zuverlässig feststellen lassen, läßt sich Naloxon insbesondere in Arzneimitteln, die weitere Hilfs- und Wirkstoffe enthalten, hervorragend und sicher stabilisieren.

Gegenstand der vorliegenden Erfindung ist daher ein Verfahren zur Stabilisierung von Naloxon und von dessen Salzen, insbesondere in flüssigen oder festen Arzneimittelformen, dadurch gekennzeichnet, daß man einen organischen oder anorganischen Stabilisator, insbesondere Schwefeldioxid, schweflige Säure oder deren Alkali- oder Erdalkalisalze oder Ethylendiamintetraessigsäure oder deren Salze in einer Menge zugibt, welche die Dimerisierung des Naloxons zu Bisnaloxon verhindert. In der Regel reicht von den oben beschriebenen Stabilisatoren eine Menge von 0,001 bis 1 Gew.%, bezogen auf die Gesamtmasse des Naloxon-Stoffgemisches, aus. Besonders bevorzugte Stabilisatoren sind Schwefeldioxid, bzw. schweflige Säure und deren pharmakologisch verträgliche pharmakologisch verträgliche Salze, insbesondere deren Alkali- oder Erdalkalisalze und Ethylendiamintetraessigsäure oder deren Salze. Bei vorzugsweise wässrigen Lösungen wird der Stabilisator möglichst in wasserlöslicher Form z.B. als Salz zugegeben. In festen oder halbfesten Arzneimittel formen sollte der Stabilisator fein dispergiert werden, um sicherzustellen, dass er in möglichst enger Verbindung mit dem Naloxon seine Schutzwirkung voll entfalten kann.

Ein weiterer Gegenstand der Erfindung sind feste, halbfeste oder flüssige Arzneimittelform, ausgenommen
a) transdermale therapeutische Systeme (TTS) und
b) Methyl- und Propylparaben enthaltende parenterale Zubereitungsformen,
c) semipermeable Zweischicht Zubereitungen gemäß den Ansprüchen 9 und 10,
enthaltend Naloxon oder ein pharmakologisch annehmbares Salz des Naloxons, dadurch gekennzeichnet, daß die Arzneimittelform mindestens einen der folgenden, die Dimerisierung des Naloxons verhindernden Stabilisatoren in einer Menge von 0,001 bis 5 Gew.-%, bezogen auf die Gesamtmasse des Stoffgemisches enthält:
Schwefeldioxid, Natriumsulfit, Natriumbisulfit, Sulfite, Bisulfite und Hydrogensulfite von Alkali- Erdalkali- und anderen Metallen, PHB-Ester, BHA, BHT, Gallate sowie niedere Fettsäuren, Fruchtsäuren, Phosphorsäuren, Sorbin- und Benzoesäure sowie deren Salze, Ester, Derivate und isomere Verbindungen, Lecithine, ein- und mehrfach hydroxylierte Benzolabkömmlinge, Ethylendiamintetraessigsäure und deren Salze, Citraconsäure, Cystein, L-Cystin, Conidendrine, Diäthylcarbonate, Methylendioxyphenole, Kephaline, β-β - Dithiopropionsäure, Biphenyl und andere Phenylderivate.

Die nicht vorveröffentlichte Entgegenhaltung WO 97/33566 betrifft ausschließlich feste Zweischichten-Zubereitungen von Opioiden, die u. a. auch Naloxon und bestimmte Antioxidantien enthalten können. Dem wurde bezüglich fester bzw. halbfester Zubereitungen durch entsprechenden Disclaimer (b) - Anspruch 9) bzw. Beschränkung auf flüssige Zubereitungen (Anspruch 10) Rechnung getragen.

Im folgenden wird die vorliegende Erfindung anhand von Beispielen näher erläutert.

### Beispiel 1

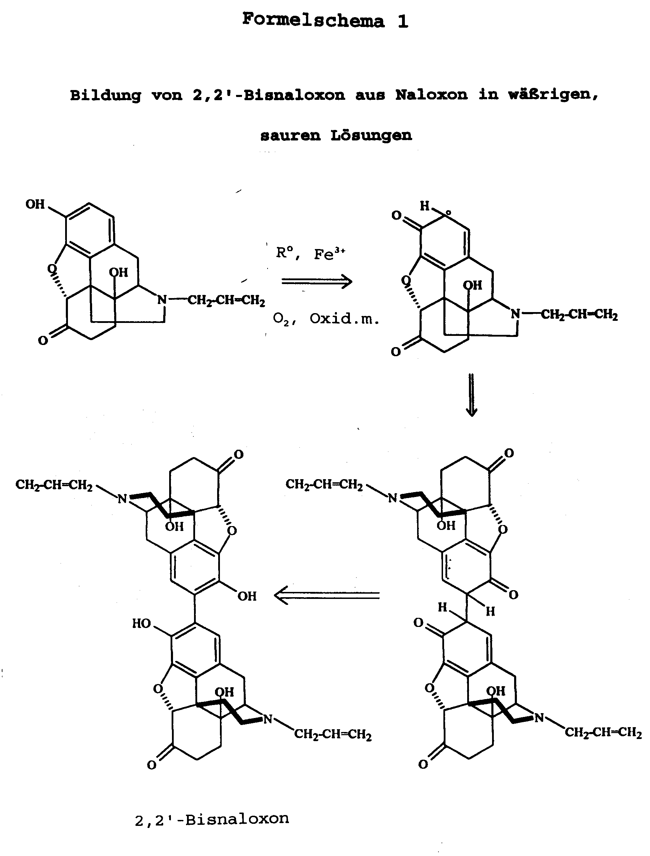

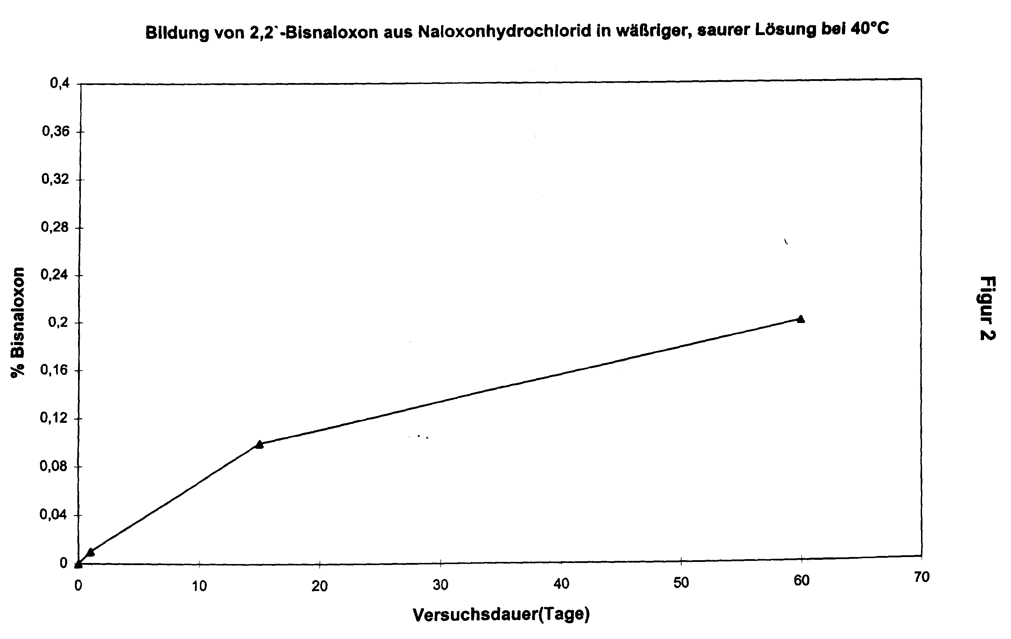

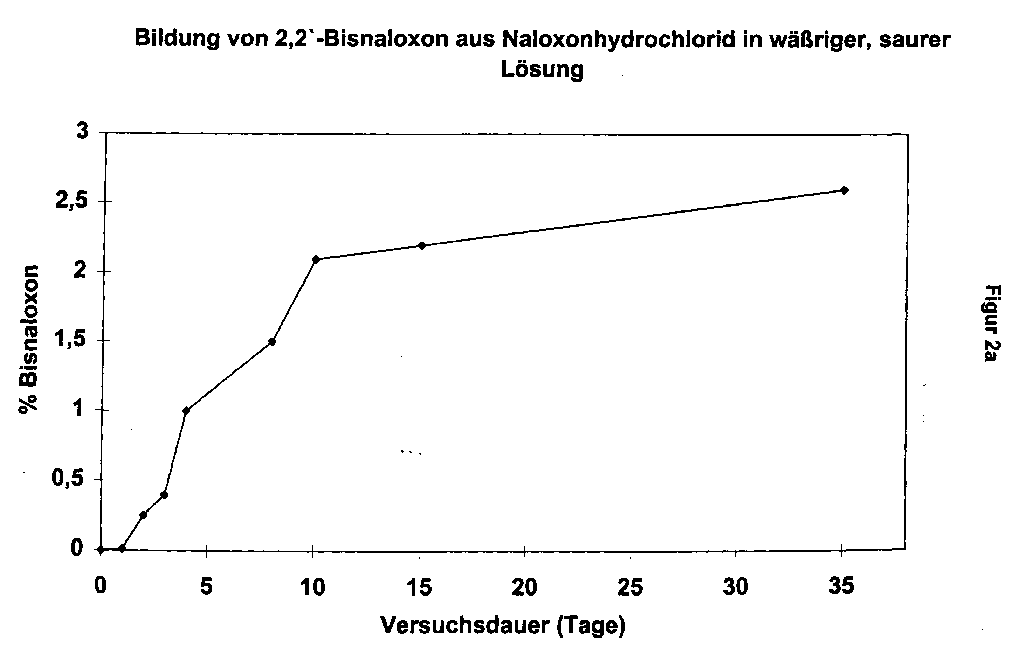

61,15 mg Naloxonhydrochlorid werden in 10 ml destilliertem Wasser gelöst. Die Lösung wird in einem verschlossenen Glasfläschchen bei 40°C gelagert. Diese Lagertemperatur entspricht der in den ICH-Richtlinien zur Stabilitätsprüfung von Arzneimitteln vorgeschriebenen Temperatur für Streßstabilitätsprüfungen. Nach 15 Tagen bzw. 2 Monaten wird der Bisnaloxongehalt der Lösung mittels HPLC bestimmt. Figur 2 zeigt, daß der Bisnaloxongehalt von <0.01% in der Ausgangslösung nach 2 Monaten auf 0.2% angestiegen ist.

### Beispiel 2

61,15 mg Naloxonhydrochlorid werden in 10 ml destilliertem Wasser gelöst. Die Lösung wird in einem verschlossenen Glasfläschchen auf 70°C erwärmt und die Bildung von Bisnaloxon mittels HPLC über mehrere Tage gemessen. Figur 2 zeigt, daß der Gehalt an Bisnaloxon innerhalb von 9 Tagen auf ca. 3 %, bezogen auf eingesetztes Naloxon, ansteigt.

### Beispiel 3

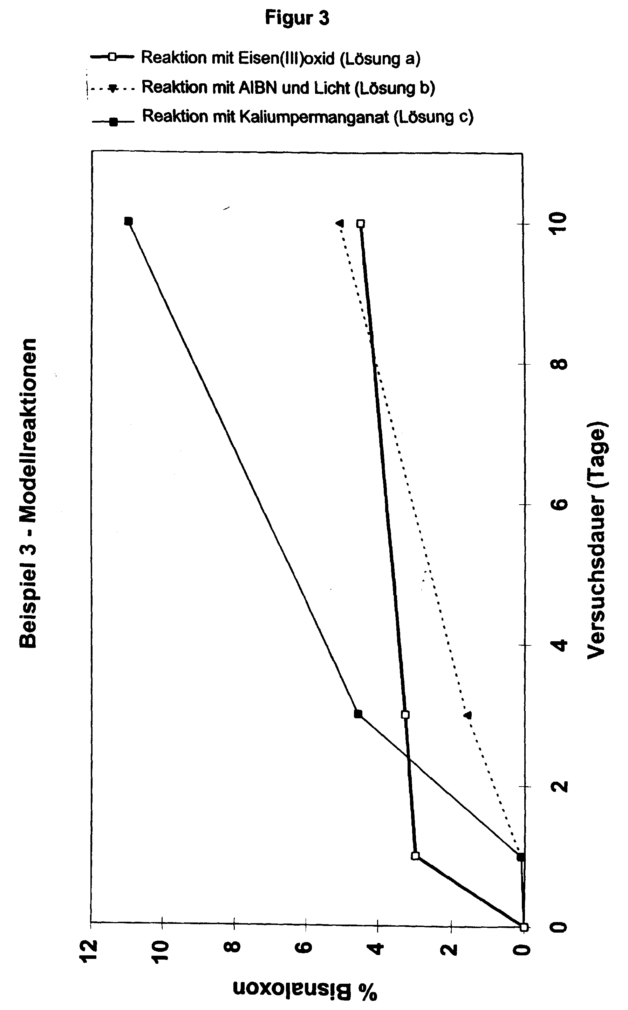

61,15 mg Naloxonhydrochlorid werden in 10 ml destilliertem Wasser gelöst. Man setzt der Lösung a) 0,8 mg Eisen(III)oxid zu (Reihe 1), einer in gleicher Weise bereiteten Naloxonhydrochloridlösung in Wasser setzt man b) 0,8 mg Azobisisobutyronitril (AIBN) (Reihe 2) und einer weiteren Lösung c) 0,85 mg Kaliumpermanganat (Reihe 3) zu. Die Lösungen werden in verschlossenen Glasfläschchen gelagert. Die Lösungen a) und c) werden bei Raumtemperatur gelagert, Lösung b) wird auch bei Raumtemperatur gelagert, zusätzlich wird die Lösung aber in einem Lichtschrank mit tageslichtähnlichem Licht bestrahlt. Figur 3 zeigt, daß sich in allen Lösungen beträchtliche Mengen von Bisnaloxon bilden.

### Beispiel 4

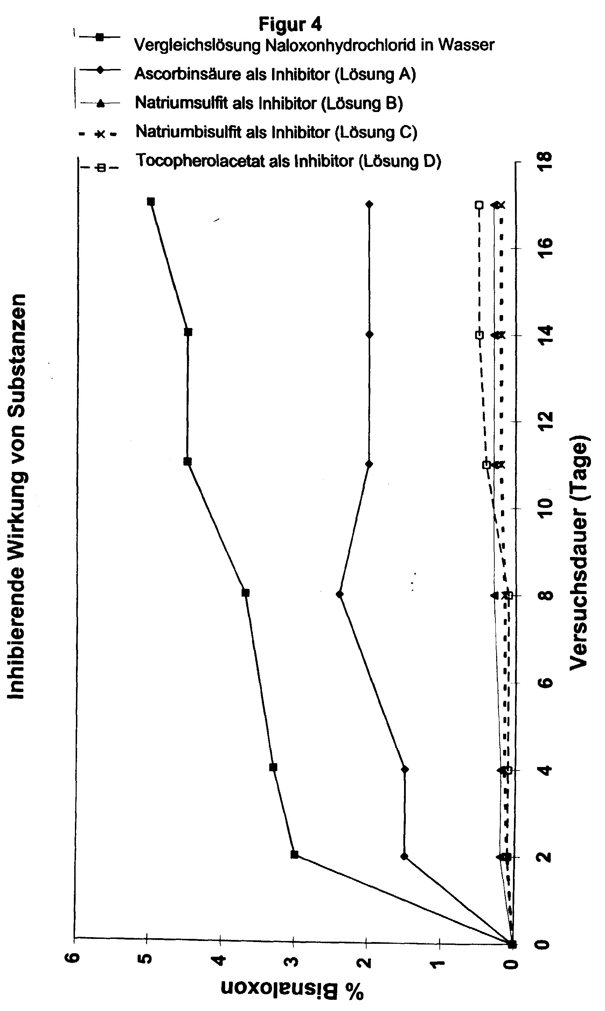

Man stellt vier Lösungen von Naloxonhydrochlorid in destilliertem Wasser, wie im Beispiel 1 beschrieben, her. Zur Lösung A werden 10,1 mg Ascorbinsäure, zur Lösung B 9,8 mg Natriumsulfit, zur Lösung C 9,5 mg Natriumbisulfit und zur Lösung D 20,6 mg Tocopherolacetat zugegeben. Die Lösungen werden in verschlossene Glasfläschchen abgefüllt und wie im Beipiel 2 beschrieben mehrere Tage auf 70°C erwärmt. Mittels chromatographischer Methoden wird die Bildung von Bisnaloxon bestimmt. Figur 4 zeigt, daß die genannten Substanzen alle einen inhibierenden Effekt besitzen. Möglicherweise ist dieser bei der Ascorbinsäure aufgrund der bekannten pH- und Temperaturlabilität der Substanz hier weniger ausgeprägt als bei den anderen verwendeten Verbindungen. Dieses Beispiel belegt, daß die vorgeschlagenen Stoffe in der Lage sind, die Bildung von Bisnaloxon in sauren Naloxonhydrochloridlösungen zu verhindern.

### Beispiel 5

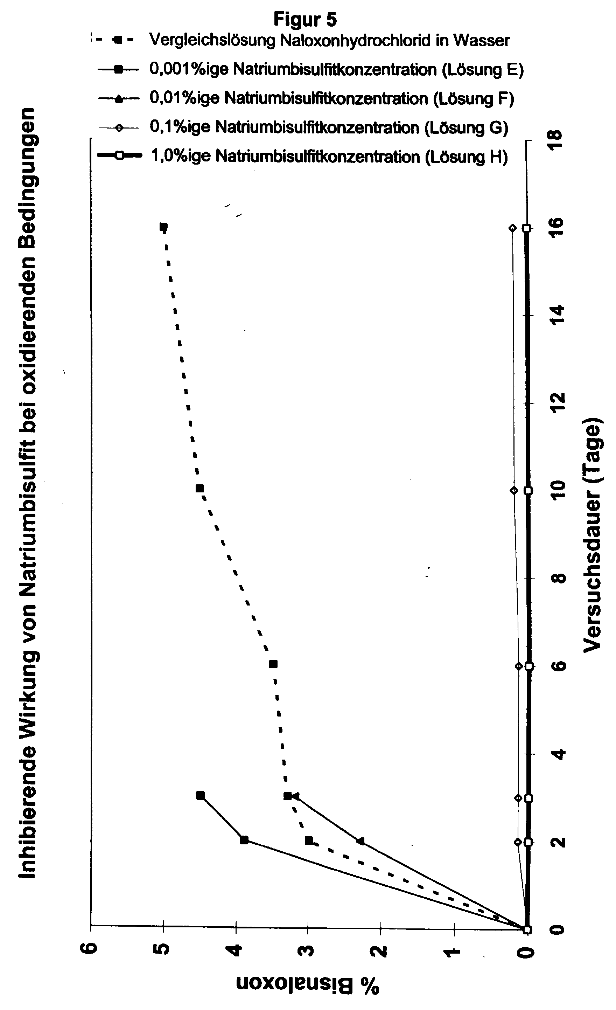

Man stellt vier Lösungen von Naloxonhydrochlorid in destilliertem Wasser, wie im Beispiel 1 beschrieben, her. Zu den Lösungen setzt man jeweils soviel Natriumbisulfit zu, daß daraus Lösungen resultieren, die 0,001 Gew% Bisulfit (=Lösung E), 0,01 Gew% Bisulfit (=Lösung F), 0,1 Gew% Bisulfit (=Lösung G) und 1 Gew% Bisulfit (=Lösung H) enthalten. Man unterwirft diese Lösungen der im Beispiel 3 unter c) genannten Modellreaktion, d.h. der Oxidation mit geringen Mengen Kaliumpermanganat bei Raumtemperatur. Figur 5 zeigt, daß in Abhängigkeit von der Bisulfitkonzentration ein unterschiedlich stark ausgeprägter inhibierender Effekt nachweisbar ist. Bisulfitkonzentrationen um und unter 0,01 % inhibieren in dieser Modellreaktion nur schwach bis gar nicht, über 0,01 % dagegen deutlich.

### Beispiel 6

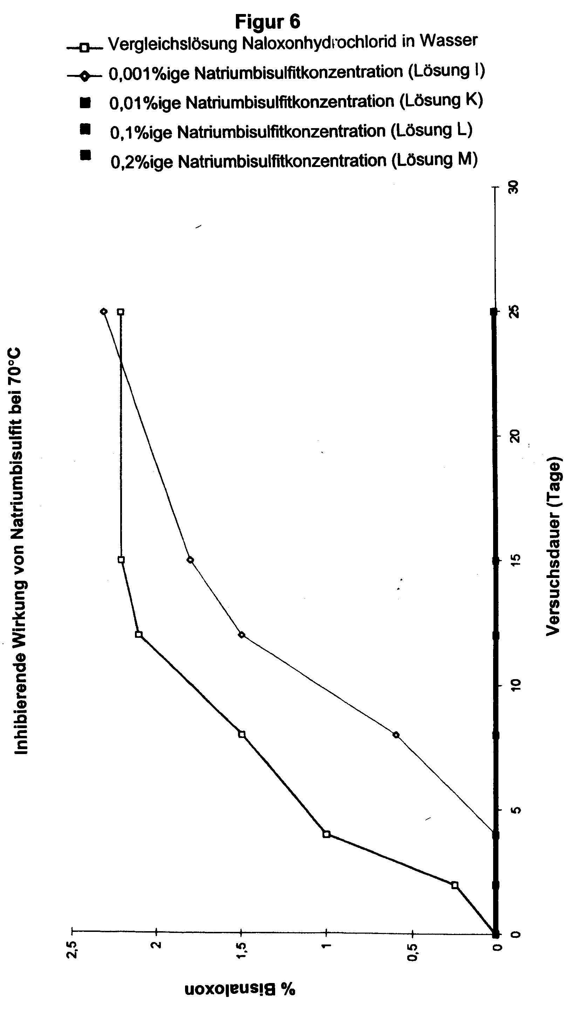

Man stellt vier Lösungen von Naloxonhydrochlorid in destilliertem Wasser, wie im Beispiel 1 genannt, her. Zu den Lösungen gibt man jeweils soviel Natriumbisulfit, daß daraus Lösungen resultieren, die 0,001 Gew% Bisulfit (=Lösung I), 0,01 Gew% Bisulfit (=Lösung K), 0,1 Gew% Bisulfit (=Lösung L) und 0,2 Gew% Bisulfit (=Lösung M) enthalten. Man unterwirft diese Lösungen der im Beispiel 2 beschriebenen Modellreaktion, d.h. erwärmt die Lösungen über mehrere Tage auf 70°C. Figur 6 zeigt, daß bei allen Bisulfitkonzentrationen ein inhibierender Effekt nachweisbar ist, der je nach Bisulfitkonzentration unterschiedlich stark ausgeprägt, aber in jedem Fall nachweisbar ist.

### Beispiel 7

Zum Nachweis der besonderen Wirksamkeit von EDTA bzw. von deren Salzen wird in diesem Beispiel auf die im DE-GBM 29 720 448.3 veröffentlichten Vergleichsdaten zurückgegriffen:

### 1. Streßtest

Formulierungen gemäß Beispiel (a) unter nachstehendem Punkt 2. mit oder ohne Zusatz von Na-EDTA (0,001 Gew.-%) wurden einerseits in einem Metallgefäß und andererseits in einem Glasgefäß hergestellt und anschließend einem Streßtest (2 bzw. 5 Tage bei 60°) unterworfen. Die Ergebnisse sind in der nachstehenden Tabelle 1 dargestellt.

**Tabelle 1**

| | **Ausgangswert** | **Streßtest 2 Tage 60°C** | **Streßtest 5 Tage 60°C** |
|---|---|---|---|
| ohne Natrium-EDTA Zusatz (hergestellt in einem Metallgefäß) | 0,125 % | 2,406 % | 3.529 % |
| mit Natrium-EDTA Zusatz (hergestellt in einem Metallgefäß) | 0,156 % | 0,143 % | 0,142 % |
| Ohne Natrium-EDTA Zusatz (hergestellt in einem Glasgefäß) | 0,176 % | 2,696 % | n.b. |
| mit Natrium-EDTA Zusatz (hergestellt in einem Glasgefäß) | 0,102 % | 0,124 % | 0,119 % |

Die Ergebnisse zeigen deutlich, daß der Zusatz von NaEDTA zu einer Tilidin und Naloxon enthaltenden Formulierung eine Dimerisierung von Naloxon zu 2,2'-Bisnaloxon verhindert, und demgemäß die Lagerstabilität derartiger Arzneimittelformulierungen mit einem Zusatz an EDTA deutlich verbessert ist.

### 2. Beispiele für Formulierungen von gemäß DE-GBM 29 720 448.3 bevorzugten pharmazeutischen Zusammensetzungen

(a) 0,72 ml Lösung enthalten:

| | |
|---|---|
| Tilidin HCl 0,5 H₂O | 51,45 mg |
| Naloxon HCl H₂O | 4,40 mg |
| Ethanol 96 % | 72,10 mg |
| Salzsäure 25 % | 1,96 mg |
| Natrium-EDTA (Dinatrium-Salz) | 0,007 mg (entspr. 0,00098 Gew.-%) |
| Gereinigtes Wasser | 587,21 mg |
| | 717,127 mg |

(b) 1,0 ml Lösung enthalten:

| | |
|---|---|
| Tilidin HCl 0,5 H₂O | 71,458 mg |
| Naloxon HCl H₂O | 6,080 mg |
| Natriumbenzoat | 1,20 mg |
| Salzsäure 25 % | 4,75 mg |
| Natrium-EDTA (Dinatrium-Salz) | 0,015 mg (entspr. 0,0014 Gew.-%) |
| Gereinigtes Wasser | 927,9865 mg |
| | 1011,476 mg |

## Patentansprüche

1. Verfahren zur Stabilisierung von Naloxon und von dessen Salzen insbesondere in flüssigen oder festen Arzneimittelformen, dadurch gekennzeichnet, daß man einen organischen oder anorganischen Stabilisator in einem Menge zugibt, welche die Dimerisierung des Naloxons zu 2,2'-Dinaloxon inhibiert.

2. Verfahren gemäß Anspruch 1, dadurch gekennzeichnet, daß als Stabilisator mindestens eine Verbindung aus der Gruppe:
Schwefeldioxid, Natriumsulfit, Natriumbisulfit, Ascorbinsäure und deren Derivate und Tocopherol sowie dessen wasser- und fettlöslicher Derivate wie z. B` Tocofersolan® oder Tocopherolacetat, Sulfite, Bisulfite und Hydrogensulfite von Alkali- Erdalkali- und anderen Metallen, PHB-Ester, BHA, BHT, Gallate sowie niedere Fettsäuren, Fruchtsäuren, Phosphorsäuren, Sorbin- und Benzoesäure sowie deren Salze, Ester, Derivate und isomere Verbindungen, Ascorbylpalmitat, Lecithine, ein- und mehrfach hydroxylierte Benzolabkömmlinge, Ethylendiamintetraessigsäure und deren Salze, Citraconsäure, Cystein, L-Cystin, Conidendrine, Diäthylcarbonate, Methylendioxyphenole, Kephaline, β,β'-Dithiopropionsäure, Biphenyl und andere Phenylderivate gewählt wird.

3. Verfahren gemäß Anspruch 1 oder 2, dadurch gekennzeichnet, daß als Stabilisator Schwefeldioxid, schweflige Säure oder deren Alkali- oder Erdalkalisalze verwendet werden.

4. Verfahren gemäß Anspruch 1 oder 2, dadurch gekennzeichnet, daß als Stabilisator Ethylendiamintetraessigsäure oder Salze derselben verwendet werden.

5. Verfahren gemäß Anspruch 1 bis 4, dadurcn gekennzeichnet, daß die zu stabilisierenden Arzneimittelformen weitere Hilfs- und Wirkstoffe enthalten.

6. Verfahren gemäß Anspruch 1 bis 5, dadurch gekennzeichnet, daß der Stabilisator in einer Menge von 0,001 bis 5 Gew.-%, bezogen auf die Gesamtmasse der Arzneimittelform verwendet wird.

7. Verfahren gemäß Anspruch 1 bis 6, dadurch gekennzeichnet, daß der Stabilisator in einer Menge von 0,001 bis 1 Gew,-%, bezogen auf die Gesamtmasse der Arzneimittelform verwendet wird.

8. Verfahren gemäß Anspruch 1 bis 7, dadurch gekennzeichnet, das der Stabilisator in einer Menge von 0,001 bis 0,5 Gew.-%, bezogen auf die Gesamtmasse der Arzneimittelform eingesetzt wird.

9. Feste oder halbfeste Arzneimittelform, ausgenommen
a) transdermale therapeutische Systeme (TTS)
b) semipermeable Zweischicht-Zubereitungen,
enthaltend Naloxon oder ein pharmakologisch annehmbares Salz des Naloxons, dadurch gekennzeichnet, daß die Arzneimittelform mindestens einen, die Dimerisierung des Naloxons verhindernden Stabilisator in einer Menge von 0,001 bis 5 Gew.-%` bezogen auf die Gesamtmasse des Stoffgemisches enthält.

10. Flüssige Arzneimittelform, ausgenommen
a) transdermale therapeutische Systeme (TTS)
b) Methyl- und Propylparaben enthaltende parenterale Zubereitungsformen
enthaltend Naloxon oder ein pharmakologisch annehmbares Salz des Naloxons, dadurch gekennzeichnet, daß die Arzneimittelform mindestens einen die Dimerisierung des Naloxons verhindernden Stabilisator in einer Menge von 0,001 bis 5 Gew.%, bezogen auf die Gesamtmasse des Stoffgemischs enthält.

11. Arzneimittelform gemäß Anspruch 9 oder 10, dadurch gekennzeichnet, das als Stabilisator mindestens eine Verbindung aus der Gruppe
Schwefeldioxid, Natriumsulfit, Natriumbisulfit, Ascorbinsäure und deren Derivate und Tocopherol sowie dessen wasser- und fettlöslicher Derivate wie z. B. Tocofersolan® oder Tocopherolacetat, Sulfite, Bisulfite und Hydrogensulfite von Alkali- Erdalkali- und anderen Metallen, PHB-Ester, BHA, BHT, Gallate sowie niedere Fettsäuren, Fruchtsäuren, Phosphorsäuren, Sorbin- und Benzoesäure sowie deren Salze, Ester, Derivate und isomere Verbindungen, Ascorbylpalmitat, Lecithine, ein- und mehrfach hydroxylierte Benzolabkömmlinge, Ethylendiamintetraessigsäure und deren Salze, Citraconsäure, Cystein, L-Cystin, Conidendrine, Diäthylcarbonate, Methylendioxyphenole, Kephaline, β,β'-Dithiopropionsäure, Biphenyl und andere Phenylderivate gewählt wird.

12. Arzneimittelform gemäß Anspruch 9 oder 10, dadurch gekennzeichnet, daß diese weitere Hilfs- und Wirkstoffe enthält.

13. Arzneimittelform gemäß Anspruch 9 bis 12, dadurch gekennzeichnet, daß diese mindestens einen Stabilisator in einer Menge von 0,001 bis 1 Gew.-%, bezogen auf die Gesamtmasse der Arzneimittelform enthält.

14. Arzneimittelform gemäß Anspruch 9 bis 13, dadurch gekennzeichnet, daß diese mindestens eigen Stabilisator in einer Menge von 0,001 bis 0,5 Gew.-%, bezogen auf die Gesamtmasse der Arzneimittelform enthält

15. Arzneimittelform gemäß Anspruch 9 bis 14, dadurch gekennzeichnet, das diese als Stabilisator Schwefeldioxid, schweflige Säure oder deren Alkali- oder Erdalkalisalze enthält.

16. Arzneimittelform gemäß Anspruch 9 bis 15, dadurch gekennzeichnet, das diese als Stabilisator Ethylendiamintetraessigsäure oder ein Salz derselben enthält.

17. Verwendung von mindestens einer Substanz aus der Gruppe:
Schwefeldioxid, Natriumsulfit, Natriumbisulfit, Ascorbinsäure und deren Derivate und Tocopherol sowie dessen wasser- und fettlöslicher Derivate wie z. B. Tocofersolan® oder Tocopherolacetat, Sulfite, Bisulfite und Hydrogensulfite von Alkali- Erdalkali- und anderen Metallen, PHB-Ester, BHA, BHT, Gallate sowie niedere Fettsäuren, Fruchtsäuren, Phosphorsäuren, Sorbin- und Benzoesäure sowie deren Salze, Ester, Derivate und isomere Verbindungen, Ascorbylpalmitat, Lecithine, ein- und mehrfach hydroxylierte Benzolabkömmlinge, Ethylendiamintetraessigsäure und deren Salze, Citraconsäure, Cystein, L-Cystin, Conidendrine, Diäthylcarbonate, Methylendioxyphenole, Kephaline, ß,ß'-Dithiopropionsäure, Biphenyl und andere Phenylderivate zur Verhinderung der Dimerisierung von Naloxon.

18. Verwendung einer Substanz gemäß Anspruch 17 zur Verhinderung der Dimerisierung von Naloxon, dadurch gekennzeichnet, daß die Substanz Schwefeldioxid oder schweflige Säure oder ein Alkali- oder Erdalkalisalz derselben ist.

19. Verwendung einer Substanz gemäß Anspruch 17 zur Verhinderung der Dimerisierung von Naloxon, dadurch gekennzeichnet, daß die Substanz Ethylendiamintetraessigsäure oder ein Salz derselben ist.

20. Verwendung einer Substanz gemäß Anspruch 17 bis 19, dadurch gekennzeichnet, daß Verhinderung der Dimerisierung in einer flussigen oder festen Arzneimittelform stattfindet.

21. Verwendung gemäß Anspruch 20, dadurch gekennzeichnet, daß die Arzneimittelform weitere Hilfs- und Wirkstoffe enthält.

## Claims

1. Process for the stabilisation of naloxone and of its salts, especially in liquid or solid medicinal forms, characterised in that one adds an organic or inorganic stabiliser in an amount which inhibits the dimerisation of the naloxone to 2,2'-dinaloxone.

2. Process according to claim 1, characterised in that, as stabiliser, at least one compound is chosen from the group:
sulphur dioxide, sodium sulphite, sodium bisulphite, ascorbic acid and its derivatives and tocopherol, as well as its water- and fat-soluble derivatives, such as e.g. tocofersolan® or tocopherol acetate, sulphites, bisulphites and hydrogen sulphites of alkali metal, alkaline earth metal and other metals, PHB esters, BHA, BHT, gallates, as well as lower fatty acids, fruit acids, phosphoric acids, sorbic and benzoic acids, as well as their salts, esters, derivatives and isomeric compounds, ascorbyl palmitate, lecithins, mono- and polyhydroxylated benzene derivatives, ethylenediamine-tetraacetic acid and its salts, citraconic acid, cysteine, L-cystine, conidendrine, diethyl carbonate, methylenedioxyphenols, kephalines, β,β'-dithiopropionic acid, biphenyl and other phenyl derivatives.

3. Process according to claim 1 or 2, characterised in that sulphur dioxide, sulphurous acid or its alkali metal or alkaline earth metal salts are used as stabiliser.

4. Process according to claim 1 or 2, characterised in that ethylenediamine-tetraacetic acid or salts thereof are used as stabiliser.

5. Process according to claim 1 to 4, characterised in that the medicament forms to be stabilised contain further adjuvant and active materials.

6. Process according to claim 1 to 5, characterised in that the stabiliser is used in an amount of 0.001 to 5 wt.%, referred to the total weight of the medicament form.

7. Process according to claim 1 to 6, characterised in that the stabiliser is used in an amount of 0.001 to 1 wt.%, referred to the total weight of the medicament form.

8. Process according to claim 1 to 7, characterised in that the stabiliser is used in an amount of 0.001 to 0.5 wt.%, referred to the total weight of the medicament form.

9. Solid or semi-solid medicament forms excluding
a) transdermal therapeutic systems (TTS) and
b) semipermeable two-layer preparations
containing naloxone or a pharmacologically acceptable salt of naloxone, characterised in that the medicament form contains at least one stabiliser preventing the dimerisation of the naloxone in an amount of 0.001 to 5 wt.%, referred to the total weight of the material mixture.

10. Liquid medicament forms excluding:
a) transdermal therapeutic systems (TTS) and
b) methyl- and propylparabene-containing parenteral forms of composition
containing naloxone or a pharmacologically acceptable salt of naloxone, characterised in that the medicament form contains at least one stabiliser preventing the dimerisation of the naloxone in an amount of 0.001 to 5 wt.%, referred to the total weight of the material mixture.

11. Medicament form according to claim 9 or 10, characterised in that, as stabiliser, at least one compound of the group:
sulphur dioxide, sodium sulphite, sodium bisulphite, ascorbic acid and its derivatives and tocopherol, as well as its water- and fat-soluble derivatives, such as e.g. Tocofersolan® or tocopherol acetate, sulphites, bisulphites and hydrogen sulphites of alkali metals, alkaline earth metals and other metals, PHB esters, BHA, BHT, gallates, as well as lower fatty acids, fruit acids, phosphoric acids, sorbic and benzoic acid, as well as their salts, esters, derivatives and isomeric compounds, ascorbyl palmitate, lecithins, mono- and polyhydroxylated benzene derivatives, ethylenediaminetetraacetic acid and its salts, citraconic acid, cysteine, L-cystine, conidendrine, diethyl carbonate, methylenedioxyphenols, kephalins, P,P'-dithiopropionic acid, biphenyl and other phenyl derivatives is chosen

12. Medicament form according to claim 9 or 10, characterised in that it contains at least further adjuvant or active materials.

13. Medicament form according to claim 9 to 12, characterised in that it contains at least one stabiliser in an amount of 0.001 to 1 wt.%, referred to the total weight of the medicament form.

14. Medicament form according to claim 9 to 13, characterized in that it contains at least one stabilizer in an amount of 0.001 to 0.5 wt.%, referred to the total weight of the medicament form.

15. Medicament form according to claim 9 to 14, characterised in that this contains sulphur dioxide, sulphurous acid or its alkali metal or alkaline earth metal salts as stabiliser.

16. Medicament form according to claim 9 to 14, characterised in that this contains ethylenediaminetetraacetic acid or a salt thereof as stabiliser.

17. Use of at least one substance from the group: sulphur dioxide, sodium sulphite, sodium bisulphite, ascorbic acid and its derivatives and tocopherol, as well as its water- and fat-soluble derivatives, such as e.g. Tocofersolan® and tocopherol acetate, sulphites, bisulphites and hydrogen sulphites of alkali metals, alkaline earth metals and other metals, PHB esters, BHA, BHT, gallates, as well as lower fatty acids, fruit acids, phosphoric acids, sorbic and benzoic acid, as well as their salts, esters, derivatives and isomeric compounds, ascorbyl palmitate, lecithins, mono- and polyhydroxylated benzene derivatives, ethylenediaminetetraacetic acid and its salts, citraconic acid, cysteine, L-cystine, conidendrine, diethyl carbonate, methylenedioxyphenols, kephalins, β,β'-dithiopropionic acid, biphenyl and other phenyl derivatives for the prevention of the dimerisation of naloxone.

18. Use of a substance according to claim 17 for the prevention of the dimerisation of naloxone, characterised in that the substance is sulphur dioxide, sulphurous acid or an alkali metal or alkaline earth metal salt thereof.

19. Use of a substance according to claim 17 for the prevention of the dimerisation of naloxone, characterized in that the substance is ethylenediaminetetraacetic acid or a salt thereof.

20. Use of a substance according to claim 17 to 18, characterised in that prevention of the dimerisation takes place in a liquid or solid medicament form.

21. Use of claim 20, characterised in that the medicament form contains further adjuvant and active materials.

## Revendications

1. Procédé de stabilisation du naloxon et de ses sels, en particulier dans des compositions pharmaceutiques liquides ou solides caractérisé en ce que l'on ajoute un stabilisant organique ou inorganique qui empêche la dimérisation du naloxon en 2,2'-dinaloxon.

2. Procédé selon la revendication 1, caractérisé en ce que, en tant que stabilisant, on utilise au moins un composé choisi dans le groupe comprenant dioxyde de soufre, sulfite de sodium, bisulfite de sodium, acide ascorbique et leurs dérivés et tocophérol ainsi que ses dérivés hydrosolubles et liposolubles comme, par exemple, le Tocofersolan® ou l'acétate de tocophérol, le sulfite, le bisulfite et l'hydrogénosulfite de métaux alcalins ou alcalino-terreux et autres métaux, esters de PHB, BHA, BHT, galate ainsi que les acides gras inférieurs, acides de fruits, acide du phosphore, acide sorbique et acide benzoïque ainsi que leurs sels, esters, dérivés et isomères, palmitate d'ascorbyle, lécithine, dérivés de la série du benzène mono ou polyhydroxylé, acide éthylènediamine tétraacétique et ses sels, acide citraconique, cystéine, L-cystérine, conidendrine, carbonate de diéthyle, méthylène de dioxyphénol, céphaline, acide β,β-dithiopropionique, biphényle et autres dérivés phényliques.

3. Procédé selon l'une quelconque des revendications 1 ou 2, caractérisé en ce que, en tant que stabilisant, on utilise du dioxyde de soufre, de l'acide sulfitique ou leurs sels alcalins ou alcalino-terreux.

4. Procédé selon l'une quelconque des revendications 1 ou 2, caractérisé en ce que, en tant que stabilisant, on utilise l'acide éthylènediamine tétraacétique ou ses sels.

5. Procédé selon l'une quelconque des revendications 1 à 4, caractérisé en ce que les médicaments à stabiliser contiennent d'autres adjuvants et produits actifs.

6. Procédé selon l'une quelconque des revendications 1 à 5, caractérisé en ce que le stabilisant est utilisé en une quantité de 0,001 à 5% en poids, exprimée par rapport à la masse totale de la composition pharmaceutique.

7. Procédé selon l'une quelconque des revendications 1 à 6, caractérisé en ce que le stabilisant est utilisé en une quantité de 0,001 à 1% en poids, exprimée par rapport à la masse totale de la composition pharmaceutique.

8. Procédé selon l'une quelconque des revendications 1 à 7, caracterisé en ce que le stabilisant est utilisé en une quantité de 0,001 à 0,5% en poids, exprimée par rapport à la masse totale de la composition pharmaceutique.

9. Compositions pharmaceutiques solides ou semi-solides à l'exception
a) des systémes thérapeutiques transdermiques (TTS); et
b) des preparations à deux enduits semiperméables,
renfermant du naloxon ou un sel pharmaceutiquement acceptable de naloxon, caractérisées en ce que la composition pharmaceutique contient a moins quantité de 0,001 a 5% en poids, exprimée par rapport à la masse totale du mélange.

10. Compositions pharmaceutiques liquides à l'exception
a) des systémes thérapeutiques transdermiques (TTS); et
b) des préparations pour administration parentérale contenant du méthylparabène et du propylparabène,
renfermant du naloxon ou un sel pharmaceutiquement acceptable de naloxon, caractérisées en ce que la composition pharmaceutique contient un stabilisant, empêchant la dimérisation du naloxon en une quantité de 0,001 a 5% en poids, exprimée par rapport à la masse totale du mélange. Composition pharmaceutique selon la revendication 9, caractérisé ce qu'elle comprend en outre d'autres adjuvants et produits actifs.

11. Composition pharmaceutique selon l'une quelconque des revendications 9 ou 10, caractérisée en ce que, en tant que stabilisant, elle contient une substance choisie dans le groupe formé par: dioxyde de soufre, sulfite de sodium, bisulfite de sodium, acide ascorbique et leurs dérivés et tocophérol ainsi que ses dérivés hydrosolubles et liposolubles comme, par exemple, le Tocofersolan® ou l'acétate de tocophérol, le sulfite, le bisulfite et l'hydrogénosulfite de métaux alcalins ou alcalino-terreux et autres métaux, esters de PHB, BHA, BHT, galate ainsi que les acides gras inférieurs, acides de fruits, acide du phosphore, acide sorbique et acide benzoïque ainsi que leurs sels, esters, dérivés et isomères, palmitate d'ascorbyle, lécithine, dérivés de la série du benzène mono ou polyhydroxylé, acide éthylènediamine tétraacétique et ses sels, acide citraconique, cystéine, L-cystérine, conidendrine, carbonate de diéthyle, méthylène de dioxyphénol, céphaline, acide β,β-dithiopropionique, biphényle et autres dérivés phényliques.

12. Composition pharmaceutique selon la revendication 9 ou 10, caractérisée en ce que la composition pharmaceutique contient d'autres adjuvants et produits actifs.

13. Composition pharmaceutique selon la revendication 9 à 12, caractérisée en ce qu'elle contient au moins un stabilisant en une quantité de 0.001 à 1% en poids, exprimée par rapport à la masse totale de la composition pharmaceutique.

14. Composition pharmaceutique selon la revendication 9 à 13, caractérisée en ce qu'elle contient au moins un stabilisant en une quantité de 0.001 à 0.5% en poids, exprimée par rapport à la masse totale de la composition pharmaceutique.

15. Composition pharmaceutique selon la revendication 9 à 14, caractérisée en ce que, en tant que stabilisant, elle contient du dioxyde de soufre, de l'acide sulfitique ou leurs sels de metaux alcalins ou alcalino-terreux.

16. Composition pharmaceutique selon la revendication 9 à 15, caractérisée en ce que, en tant que stabilisant, elle contient de l'acide éthylènediamine tétraacétique ou ses sels.

17. Utilisation au moins d'une substance choisie dans le groupe: dioxyde de soufre, sulfite de sodium, bisulfite de sodium, acide ascorbique et leurs dérivés et tocophérol ainsi que ses dérivés hydrosolubles et liposolubles comme, par exemple, le Tocofersolan® ou l'acétate de tocophérol, le sulfite, le bisulfite et l'hydrogénosulfite de métaux alcalins ou alcalino-terreux et autres métaux, esters de PHB, BHA, BHT, galate ainsi que les acides gras inférieurs, acides de fruits, acide du phosphore, acide sorbique et acide benzoïque ainsi que leurs sels, esters, dérivés et isomères, palmitate d'ascorbyle, lécithine, dérivés de la série du benzène mono ou polyhydroxylé, acide éthylènediamine tétraacétique et ses sets, acide citraconique, cystéine, L-cystérine, conidendrine, carbonate de diéthyle, méthylène de dioxyphénol, céphaline, acide β,β-dithiopropionique, biphényle et autres dérivés phényliques pour empêcher la dimérisation du naloxon.

18. Utilisation d'une substance selon la revendication 17 pour empêcher la dimérisation du naloxon, caractérisée en ce que la substance est le dioxyde de soufre ou l'acide sulfitique ou un de ses sels alcalin ou alcalino-terreux.

19. Utilisation d'une substance selon la revendication 17 pour empêcher la dimérisation du naloxon, caractérisée en ce que la substance est l'acide éthylènediamine tétraacétique ou ses sels.

20. Utilisation d'une substance selon l'une quelconque des revendications 17 à 19, caractérisée en ce que, l'inhibition de la dimérisation a lieu dans une composition pharmaceutique liquide ou solide.

21. Utilisation selon la revendication 20, caractérisée en ce que la composition pharmaceutique contient d'autres adjuvants et produits actifs.
